Europäisches Patentamt

European Patent Office

Office européen des brevets

(11) Numéro de publication : **0 632 018 A1**

# (12) DEMANDE DE BREVET EUROPEEN

(21) Numéro de dépôt : **94401446.3**

(22) Date de dépôt : **27.06.94**

(51) Int. Cl.[6] : **C07C 275/02,** C07C 273/02, C05F 7/00, C05C 9/00

(30) Priorité : **29.06.93 FR 9308044**

(43) Date de publication de la demande :
**04.01.95 Bulletin 95/01**

(84) Etats contractants désignés :
**BE DE ES GB GR IT NL**

(71) Demandeur : **INSTITUT FRANCAIS DU PETROLE**
**4, Avenue de Bois Préau**
**F-92502 Rueil-Malmaison (FR)**

(71) Demandeur : **TRANSVALOR**
**60, Boulevard Saint-Michel**
**F-75272 Paris Cédex 06 (FR)**

(72) Inventeur : **Lonchamp, Daniel**
**27 Montée de Verdun**
**F-69160 Tassin la Demi-Lune (FR)**
Inventeur : **Schwob, Yvan**
**14, Allée du Parc Saint Jean**
**F-06400 Cannes (FR)**

(54) **Composition sulfo-uréique résultant de l'action de l'urée sur une boue sulfurique d'origine pétrolière, procédé de préparation et utilisation comme source de matière fertilisante.**

(57) Composition sulfo-uréïque formée par le produit résultant de l'action de l'urée sur une boue d'origine pétrolière contenant en poids de 50 à 99% d'acide sulfurique et de 1 à 20% de résidus organiques. La boue acide peut contenir de l'eau et provient souvent d'une réaction d'alkylation. Cette composition est utilisable comme source de matière fertilisante ou comme composant d'une formulation fertilisante.

EP 0 632 018 A1

La présente invention concerne un procédé pour le traitement et la valorisation de boues sulfuriques d'origine pétrolière consistant essentiellement à faire réagir lesdites boues sur de l'urée dans des proportions définies de manière à engager l'acide sulfurique dans une composition sulfo-uréique particulière. Elle concerne également la composition sulfo-uréïque résultant dudit procédé. Le procédé permet de récupérer l'acide sulfurique en vue de sa valorisation de même qu'une partie des composés organiques présents dans les boues.

L'art antérieur est notamment illustré par le brevet US-A-4 397 675 et par l'article Chem. Abstracts, vol. 113, n° 18, 29 Octobre 1990, Columbus, Ohio, US ; abstract n° 155 239 k, page 166.

Il est connu que certaines opérations chimiques notamment celles effectuées dans les processus d'alkylation. et en particulier d'alkylation aliphatique, sont réalisées en présence d'acide sulfurique concentré jouant un rôle catalytique. Malheureusement, une partie de l'acide engagé se retrouve en fin d'opération sous forme de boues sulfuriques impures dont l'élimination pose de nombreux problèmes. La présente invention permet en particulier la valorisation et le traitement de ces boues issues des réactions d'alkylations aliphatiques.

L'homme du métier sait que l'essentiel de ces boues est constitué de mélanges ou de combinaisons complexes entre l'acide sulfurique et certains résidus organiques. Leur composition moyenne en poids oscille entre les valeurs suivantes exprimées en pourcentage pondéral :

- $SO_4 H_2$        88 à 96%
- $H_2O$        2 à 6%
- résidus organiques        2 à 6%

Les demandeurs ont trouvé que le traitement de ces boues par de l'urée en proportion définie, pouvait conduire à des combinaisons sulfouréiques valorisables tandis qu'une partie des résidus organiques, sous forme d'hydrocarbures par exemple. pouvait être également récupérée.

Il est connu d'obtenir entre l'urée et l'acide sulfurique, des complexes mono et biuréiques à points de fusion bien définis. Il est connu aussi que les complexes donnent entre eux et avec l'urée des composés fusibles à basse température. C'est ainsi qu'un eutectique comprenant 3,6 moles d'urée pour une mole d'acide sulfurique se présente sous forme d'un liquide stable à une température proche de 25°C.

Les demandeurs ont trouvé de manière surprenante que l'acide sulfurique impur en provenance des opérations d'alkylation pouvait, lui aussi, permettre de fabriquer de telles combinaisons et que l'excès d'urée engagée pour ce faire conduisait à de telles combinaisons et en outre que cet excès conduisait à la libération, par une sorte de saponification, d'une partie des composés organiques préalablement bloqués par l'acide. Les boues sulfuriques peuvent être considérées comme étant un mélange d'acide, d'eau et de sulfate acide d'hydrocarbures et en particulier de sulfate acide d'alkyle. Si nous désignons par R, le ou les restes organiques liés à l'acide, le mélange peut être considéré comme suit :

$$a\, SO_4\, H_2 - b\, SO_4\, HR - c\, H_2O$$

$a$, $b$ et $c$, représentent le nombre de moles de chaque composé présent dans le mélange. On sait aussi que la présence d'une faible quantité d'eau dans ce composé peut être admise sans problème particulier.

De manière surprenante, l'action de l'urée sur l'acide sulfurique contenu dans ces boues acides se déroule selon le procédé classique d'obtention d'un composé sulfo-uréique comme c'est le cas avec de l'acide sulfurique sensiblement pur. L'action de l'urée en excès sur les sulfates acides d'hydrocarbures, conduit à une sorte de saponification en la présence d'eau et qui peut s'écrire par exemple dans le cas de sulfate acide d'alkyle :

$$SO_4\, HR + 2\, CO\, (NH_2)_2 + H_2O \rightarrow SO_4H_2\, 2CO\, (NH_2)_2 + R\text{-}OH$$

Suivant le poids moléculaire de R et les conditions opératoires (température et pression), les composés R-OH peuvent être récupérés.

Plus précisément, l'invention concerne une composition sulfouréïque qui est le produit résultant de l'action de l'urée sur une boue sulfurique d'origine pétrolière contenant en poids de 50 à 99% d'acide sulfurique et de 1 à 20% de résidus organiques. Le plus souvent la boue sulfurique d'origine pétrolière contient en outre de l'eau en une quantité représentant jusqu'à 6% en poids par rapport au poids total de la boue. Le plus souvent la boue sulfurique d'origine pétrolière contient de 70% à 98% en poids d'acide sulfurique et de 2 à 10% de résidus organiques.

La boue sulfurique résiduaire est tout d'abord analysée chimiquement de manière à déterminer avec précision sa contenance en eau, en matières organiques et en acide sulfurique. Cette dernière est déterminée par tous moyens connus, de manière à obtenir le taux d'acidité totale contenue dans la boue. Un moyen simple consiste par exemple à faire réagir une partie aliquote de boue avec un excès d'une base forte telle que la soude par exemple et de doser, en retour, l'excès subsistant après neutralisation de la boue. Grâce à l'emploi de solutions titrées, il est facile de connaître ainsi la quantité totale d'acide sulfurique présente. La quantité d'eau contenue dans la boue peut être déterminée par exemple par la méthode classique dite de Karl-Fischer.

Ce calcul étant fait, la boue est mise en réaction avec une quantité d'urée telle que le rapport molaire entre l'urée et l'acide total contenu dans la boue (tel que déterminé par le dosage) soit par exemple d'environ 1 : 1 à environ 4 : 1 et de préférence d'environ 3 : 1 à environ 4 : 1.

Dans une forme de réalisation particulière de la présente invention la quantité d'eau contenue dans la boue peut si nécessaire être ajustée par addition d'eau à la boue de manière à ce que le rapport molaire entre l'eau et l'acide total soit d'environ 1 : 1 à environ 6 : 1, et de préférence d'environ 1 : 1 à environ 4 : 1. Cette quantité d'eau peut-être ajustée en fonction de l'utilisation et du transport en phase liquide ou solide de la composition.

La réaction de l'urée sur la boue étant fortement exothermique il convient d'éliminer par tous moyens connus, les calories excédentaires de façon à limiter de préférence la température maximale du milieu au cours de la réaction à 75°C sous peine de voir une partie de l'urée se décomposer par effet Kjeldahl en sulfate d'ammoniac.

Il est ainsi possible d'opérer en discontinu ou en continu. Lorsque l'on opère en continu, il est préférable d'introduire les réactifs c'est-à-dire la boue, l'urée et si nécessaire de l'eau, dans un mélange dit sulfo-uréique obtenu lors d'une réaction discontinue précédente.

L'emploi d'urée solide présente l'avantage de fournir dans le milieu réactionnel la chaleur négative de dissolution. pratiquement égale à la chaleur de fusion. De cette manière le risque d'élévation anormale de la température est très limité et le contrôle de la température facilité.

Lorsque ces opérations sont conduites à des températures comprises entre environ 20°C et 75°C et à pression atmosphérique, un dégagement gazeux est observé. Ce gaz contient de l'anhydride sulfureux qui peut être récupéré par tous moyens connus de l'homme du métier.

Les compositions ainsi obtenues, titrant entre 15 et 31% en poids d'azote peuvent être utilement utilisées comme source de matière fertilisante, soit seules, soit après mélange en combinaison avec d'autres éléments fertilisants. Le procédé de l'invention permet ainsi de valoriser des boues acides et d'éviter leur coûteuse élimination. La présente invention concerne également l'utilisation des compositions décrites ci-devant comme composant d'une formulation fertilisante.

## Exemple 1

Une boue issue d'une unité d'alkylation sulfurique a la composition suivante exprimée en pourcentage en poids :
- acide sulfurique     90,2
- eau     4,7
- hydrocarbures     5,1

1000 (g.) grammes de cette boue d'alkylation sont versés dans un réacteur agité (1) maintenu à la température constante de 40°C par thermostatisation externe (2), (voir figure ci-après).

On ajoute lentement par la canalisation (3) 117 g. d'eau, puis au moyen de la vanne écluse (4), une partie de l'urée contenue dans le réservoir (5).

L'addition de l'urée se fait d'abord lentement pour éviter tout échauffement c'est-à-dire que la vitesse d'addition est régulée par incréments pour garder la température constante à 40°C, puis plus rapidement lorsque la quantité d'urée ajoutée correspond à la formation du complexe mono-uréique, c'est-à-dire après addition de 600 g. d'urée environ.

Au total, on ajoute 1988 g. d'urée granulée commerciale. Ces quantités sont telles que les proportions molaires urée : eau : acide sont de 3,6 : 1 : 1.

Les gaz qui se dégagent sont d'abord refroidis dans le piège (6) maintenu à -40°C puis barbotent dans le piège (7) contenant une solution d'eau oxygénée à 10% en volume.

Lorsque l'urée a été entièrement introduite dans le réacteur (1) on purge l'ensemble du dispositif par introduction d'un léger flux d'air par la canalisation (3). Ainsi, on déplace l'anhydride sulfureux pouvant encore être contenu dans le réacteur (1) dans les canalisations et dans le piège (6) vers le piège (7) dans lequel l'anhydride sulfureux se transforme en acide sulfurique. Le dosage de cet acide permet de connaître la quantité d'anhydride sulfureux dégagé. Cette quantité est de 0,91 g.

Le complexe sulfo-uréique formé dans le réacteur est un liquide brun foncé que l'on récupère par la canalisation (9) et qu'on laisse décanter plusieurs heures à température ambiante. Sa teneur pondérale en azote est de 30,3%. Il apparaît à la surface une pellicule fine d'un produit noir à l'aspect goudronneux qui durcit par refroidissement. Ce produit peut être récupéré par écrémage. Il représente 45 g.

## Exemple 2

On prépare un complexe sulfo-uréique ayant, comme dans l'exemple 1, un rapport molaire urée/$H_2SO_4$ de 3,6 mais avec un point de cristallisation de l'ordre de -10°C.

La boue d'alkylation est celle de l'exemple 1.

Le réacteur est maintenu à 25° C par thermostatisation externe.

On ajoute 537 g. d'eau lentement sous forte agitation pour éviter tout échauffement excessif.

Puis comme dans l'exemple 1 on ajoute 1988 g. d'urée granulée technique jusqu'à dissolution complète. Ces quantités sont telles que les proportions molaires urée : eau : acide sont de 3,6 : 3,5 : 1.

A température ambiante le complexe sulfo-uréique formé contient 26,3% en poids d'azote et laisse décanter une pellicule d'hydrocarbures noirâtre qui peut être éliminée par écrémage. et qui représente 44g.

**Exemple 3**

Une autre boue usée d'alkylation sulfurique a la composition suivante exprimée en pourcentage en poids.
- acide sulfurique     95,2
- eau     2,8
- hydrocarbures     2,0

Dans le réacteur décrit dans l'exemple 1 on verse 1000 g. de cette boue sulfurique et on ajoute lentement sous forte agitation 147 g. d'eau.

Le réacteur est thermostatisé à 50°C.

Puis on ajoute 2097 g. d'urée granulée en 30 minutes environ. Ces quantités sont telles que les proportions molaires urée : eau : acide sont 3,6 : 1 : 1.

L'acide sulfurique formé dans le piège (7) contenant de l'eau oxygénée à 10% en volume correspond à 0,1 g. d'anhydride sulfureux.

Le complexe sulfo-uréique liquide est laissé au repos 2 heures à 50°C puis une nuit à température ambiante. Une très fine couche d'hydrocarbures semi-solides se forme à la surface et peut être facilement enlevée. Son poids est de : 18,8 g. La teneur pondérale en azote de la composition après écrémage est de 30%.

**Exemple 4**

Avec la boue d'alkylation de l'exemple 3 on forme un complexe sulfouréique liquide à température ambiante mais dans lequel le rapport molaire urée/acide sulfurique est de 1,2. Les quantités sont telles que les proportions molaires urée: eau : acide sont de 1,2 : 1,4 : 1.

Le réacteur est maintenu à 25°C et à 1000 g. de boue d'alkylation on ajoute 217 g. d'eau et 700 g. d'urée granulée

Le complexe sulfo-uréique liquide est laissé au repos 2 heures à 50°C puis une nuit à température ambiante. Sa teneur en azote est de de 17,2%. Une très fine couche d'hydrocarbures semi-solides se forme à la surface et peut être facilement enlevée.Son poids est de : 16,7 g.

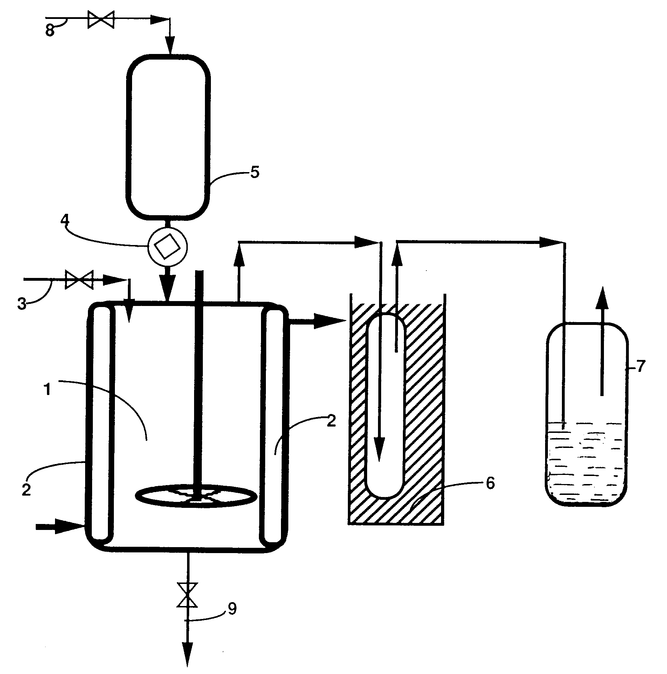

**Revendications**

**1 -** Composition sulfo-uréïque caractérisée en ce qu'elle est le produit résultant de l'action de l'urée sur une boue sulfurique d'origine pétrolière contenant en poids de 50 à 99% d'acide sulfurique et de 1 à 20% de résidus organiques.

**2 -** Composition selon la revendication 1 dans laquelle on utilise une quantité d'urée telle que le rapport molaire entre l'urée et l'acide total contenu dans la boue soit d'environ 1 : 1 à environ 4 : 1.

**3 -** Composition selon l'une des revendications précédentes dans laquelle la boue sulfurique contient en outre de l'eau en une quantité représentant jusqu'à 6% en poids par rapport au poids total de la boue.

**4 -** Composition selon l'une des revendications précédentes dans laquelle on ajoute à la boue sulfurique, une quantité d'eau telle que le rapport molaire entre l'eau et l'acide total contenu dans la boue soit d'environ 1 : 1 à environ 6 : 1.

**5 -** Composition selon l'une des revendications précédentes dans laquelle la boue sulfurique est une boue issue des réactions d'alkylations aliphatiques.

**6 -** Composition selon l'une des revendications précédentes dans laquelle l'action de l'urée sur la boue sulfurique s'effectue en en limitant la température du milieu à une valeur au plus égale à environ 75°C.

**7 -** Composition selon l'une des revendications précédentes dans laquelle l'action de l'urée sur la boue sulfurique s'effectue sous pression atmosphérique et à une température d'environ 20°C à environ 75°C.

**8 -** Procédé de préparation d'une composition fertilisante dans lequel on fait réagir, à une température au plus égale à 75°C, de l'urée, une boue sulfurique d'origine pétrolière contenant en poids 50 à 99% d'acide sulfurique et 1 à 20% de résidu organique et éventuellement de l'eau, en quantité telle que le rapport molaire entre l'urée et l'acide total contenu dans la boue soit d'environ 1 : 1 à environ 4 : 1, on décante éventuellement la solution obtenue et on récupère la composition fertilisante.

**9 -** Utilisation d'une composition selon l'une des revendications 1 à 7 comme source de matière fertilisante.

**10 -** Utilisation d'une composition selon l'une des revendications 1 à 7 comme composant d'une formulation fertilisante.

EP 0 632 018 A1

**Office européen des brevets**

# RAPPORT DE RECHERCHE EUROPEENNE

Numero de la demande

EP 94 40 1446

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | US-A-4 397 675 (D.C. YOUNG)<br>* colonne 4; exemples *<br>--- | 1 | C07C275/02<br>C07C273/02<br>C05F7/00 |
| D,A | CHEMICAL ABSTRACTS, vol. 113, no. 18,<br>29 Octobre 1990, Columbus, Ohio, US;<br>abstract no. 155239k,<br>page 166 ;<br>& CS-A-262 211 (P. KADLAS, et al.) 15 Mai<br>1989<br>* abrégé *<br>----- | 1,9 | C05C9/00 |

| DOMAINES TECHNIQUES RECHERCHES (Int.Cl.6) |
|---|
| C07C<br>C05F<br>C05C |

Le présent rapport a été établi pour toutes les revendications

| Lieu de la recherche | Date d'achèvement de la recherche | Examinateur |
|---|---|---|
| LA HAYE | 17 Août 1994 | English, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................................
& : membre de la même famille, document correspondant

EPO FORM 1503 03.82 (P04C02)

6